# EUROPEAN PATENT APPLICATION

(11) **EP 2 363 395 A2**
(43) Date of publication of application: **07.09.2011**
(21) Application number: 11156404.3
(22) Date of filing: 01.03.2011
(51) Int. Cl.: C07D 231/12, A61K 31/415

(54) **Process for preparation of celecoxib crystalline form**

(30) Priority: 01.03.2010 IN DE05282010; 17.05.2010 US 345291
(71) Applicant: Dr. Reddy's Laboratories Ltd., Hyderabad 500 016 (IN)
(72) Inventor: Muppidi, Vamsee Krishna, Andhra Pradesh 507303 (IN); Ragineni, Srinivasulu, Andhra Pradesh 509102 (IN); Duggirala, Naga Kiran, Andhra Pradesh 521230 (IN); Ramakrishnan, Srividya, Andhra Pradesh 500049 (IN); Tummala, Arjun Kumar, Andhra Pradesh 530018 (IN)
(74) Representative: Train, Matthew

(57) **Abstract**

Celecoxib crystalline Form III substantially free of celecoxib crystalline Forms I and II and a process for its preparation.

## Description

### INTRODUCTION

The present application relates to crystalline Form III of celecoxib substantially free from Form I and Form II, and a process for its preparation.

Celecoxib is described chemically as 4-[5-(4-methylphenyl)-3-(trifluoromethyl)-1H-pyrazol-1-yl]benzenesulphonamide and is structurally represented by Formula 1.

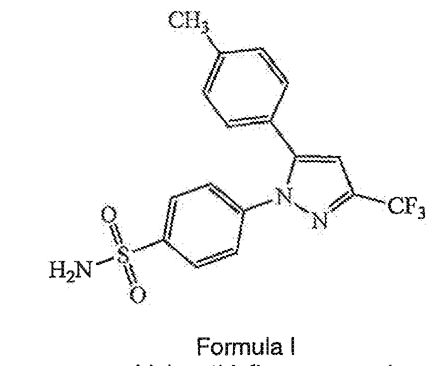

Gelecoxib is a non-steroidal anti-inflammatory drug and is indicated for the treatment of osteoarthritis, rheumatoid arthritis, juvenile rheumatoid arthritis in patients two years and older, and treatment of ankylosing spondylitis, acute pain, primary dysmenorrheal, and familial adenomatous polyposis. The drug is the active ingredient in products sold as CELEBREX™ in the United States of America.

U.S. Patent No. 5,466,283 discloses celecoxib and its pharmaceutically acceptable salts, a pharmaceutical composition and method of treatment. U.S. Patent No. 6,964,978 discloses amorphous celecoxib, which exhibits a glass transition measurable by differential scanning calorimetry. U.S. Patent No. 7,476,744 discloses crystalline Forms I, II, and III of celecoxib characterized by their X-ray powder diffraction pattern, DSC endotherm, and IR patterns. The patent also provides processes for preparation of crystalline Forms I and II by performing recrystallization at a temperature above the enantiotropic transition temperature of Form I or Form II respectively. The patent refers back to US 5,910,597 for process for preparation of polymorphic Form III in a combination of 2-propanol and water. There is no specific disclosure for preparation of polymorphic Form III either in US 7,476,744 or US 5,910,597.

U.S. Patent No. 5,466,823 discloses the recrystallization of celecoxib from diethyl ether/hexane to obtain celecoxib as a light yellow or tan solid. It also describes the recrystallization from ethyl acetate and iso-octane to obtain celecoxib as a yellow solid having a melting point of 157°C to 159°C. U.S. Patent No. 5,910,597 describes a one-pot synthesis of celecoxib and discloses the use of ethyl acetate and heptane for its recrystallization. U.S. Patent No. 6,391,906 discloses the use of a mixture of 2-propanol and water for the recrystallization of celecoxib.

None of the references in the prior art provide processes for preparation of pure celecoxib crystalline forms free from contamination with other polymorphic forms. The present application provides such a process, wherein the process provides crystalline Form III which is free from contamination with crystalline Forms I and II. The processes of the present application are robust and reproducible.

### SUMMARY OF THE INVENTION

In one aspect, the present application provides pure crystalline Form III of celecoxib, which is substantially free from crystalline Forms I and II.

In another aspect, the present application provides a process for preparation of celecoxib Form III substantially free of crystalline Forms I and II comprising:
a) providing a solution of colecoxib in an alcoholic or a nitrile solvent;
b) combining the solution of celecoxib with water; and
c) isolating pure crystalline Form III of celecoxib.

Yet another aspect of the present application relates to a process for the preparation of celecoxib Form III free of crystalline Forms I and II using a wet-milling technique.

### BRIEF DESCRIPTION OF THE DRAWING

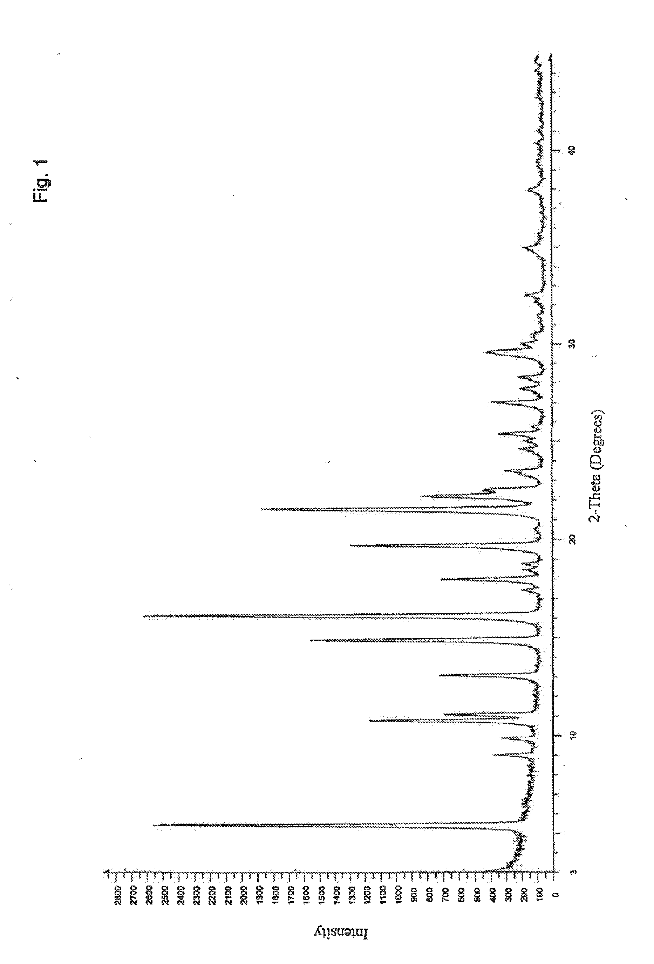

Fig. 1 is a powder X-ray diffraction ("PXRD") pattern of crystalline Form III of celecoxib, obtained as per Example I of the present application.

### DETAILED DESCRIPTION

In one aspect, the present application provides pure crystalline Form III of celecoxib, which is substantially free from crystalline Forms I and II.

In one aspect of the application, the content of the Form I impurity in Form III by powder X-ray diffractometer is determined with respect to the presence of characteristic peak at 7.1° (± 0.1°) degrees 2θ.

In one aspect of the present application, the content of the Form II impurity in Form III by the S/N ratio method is determined with respect to the presence of a characteristic peak at 13.8 (± 0.1°) degrees 2θ.

In one aspect of the application, crystalline Form III contains celecoxib Form II below the limit of detection as measured by an S/N ratio method.

In another aspect, the present application provides a process for preparation of celecoxib Form III substantially free of crystalline Forms and II comprising:
a) providing a solution of celecoxib in an alcoholic or a nitrile solvent;
b) combining the solution of celecoxib with water; and
c) isolating pure crystalline Form III of celecoxib.

Step a) involves providing a solution of celecoxib in an alcoholic or nitrile solvent. The solution of celecoxib can be obtained by dissolving celecoxib in the selected solvents. Any form of celecoxib is acceptable for forming the solution, such as any crystalline or amorphous form of celecoxib including senates with alcohols such as methanol, ethanol, or propanol, or nitriles like acetonitrile and hydrates. The solution can also be obtained directly from a reaction in which celecoxib is formed. Suitable alcoholic solvents include, but are not limited to methanol, ethanol, 2-propanol, 1-propanol, 1 -butanol, tertiary-butyl alcohol, and the like; and suitable nitrile solvents include, but are not limited to nitriles such as acetonitrile, propionitrile and the like.

Suitable temperatures for dissolving celecoxib in step a) may range from about 20°C to about the reflux temperature of the organic solvent used, such as from about 200C to about 50°C, or from about 25°C to about 40°C. The amounts of solvent used for dissolving celecoxib in step a) may range from about 2 mL to about 50 mL, per gram of celecoxib. In embodiments, the amounts of solvent may range from about 4 to about 25 mL, or from 4 to about 20 mL, per gram of colecoxib. Optionally, the solution can be given an activated charcoal treatment to remove the colored impurities or to reduce the content of metals, if any, or to remove the extraneous matter from the solution containing celecoxib.

Step b) involves combining the solution obtained in step a) with water. The step of combining with water may be performed either by adding a solution of celecoxib obtained in step a) to the water, or *vice versa.* Suitably, water is added slowly to the solution over a period of time, such as from about 30 minutes to about 4 hours depending on the batch size. The ratios of the organic solvent and water affect the efficiency of precipitation of the product. The ratio of the organic solvent and water used for the process of the present application may range from about 1:0.5 to about 1:20. In one embodiment, the ratio of the organic solvent and water may range from about 1:1 to about 1:7. In another embodiment, the ratio of the organic solvent and water may range from about 1:1 to about 1:5. Suitably, the mixture may be stirred for from about 15 minutes to about 7 hours, or longer. The mixture may be stirred for from about 20 minutes to about 5 hours, or for from about 1 hour to about 5 hours.

The mixture may be stirred at temperatures from about 10°C to about 100°C. In embodiments, the mixture may be stirred at temperatures from about 20°C to about 50°C, or from about 25°C to about 40°C.

Step c) involves isolation of crystalline Form III of celecoxib from the mixture.

Isolation can suitably be carried out using methods known in the art. The methods by which the solid material is recovered from the mixture may involve any suitable techniques, such as, for example, decantation, filtration by gravity or suction, centrifugation, and the like.

Suitably, celecoxib Form III may be isolated by filtration and, if desired, the solid may be further washed with a solvent, or water used in step b), to remove occluded mother liquor.

The obtained wet cake may be further dried using any conventional techniques, such as drying in a tray dryer, cone vacuum dryer, air oven, fluidized bed dryer, spin flash dryer, flash dryer, and the like. In embodiments, the drying may be carried out at temperatures from about 25°C to about 75°C, with or without vacuum.

Celecoxib prepared according to processes of the present application is crystalline Form III and is substantially free from crystalline Form I and Form It of celecoxib, i.e., either Form I or Form II, or both, are below detection levels.

Celecoxib, when prepared according to a process of the present application, may have a chemical purity of more than about 95%, more than about 98%, more than about 99%, or more than about 99.5%, by weight as determined using high performance liquid chromatography ("HPLC").

Celecoxib obtained as described herein may have residual solvent contents in amounts that are within the limits given by the International Conference on Harmonization of Technical Requirements for Registration of Pharmaceutical for Human Use ("ICH") guidelines. The crystalline Form III of celecoxib obtained by processes of the present application as described herein may be characterized by a PXRD pattern having peaks at about 5.4, 9.0, 9.8, 10.7, 11.0, 13.0, 14.9, 16.1, 18.0, 19.7, 21.5, 22.2 and 22.5 ± 0.2 degrees 20. The crystalline Form III of celecoxib obtained also may be characterized by a PXRD pattern as substantially depicted in Fig. 1. The PXRD patterns reported herein for celecoxib were obtained using a Brucker Axe D8 Advance Powder X-ray diffractometer, with copper Kα radiation.

Yet another aspect of the present application relates to a process for the preparation of celecoxib Form III free of crystalline Forms I and II using wet-milling technique. It has been observed that the crystalline Form III of celecoxib is sensitive to milling operation. Hence sufficient care must be taken during the milling operation for preventing conversion of the crystalline Form III to other polymorphic forms. The inventors have observed that wet-milling of the material helps retain the polymorphic form of the material, and also provides celecoxib Form III which is free from polymorphic Forms I and II.

The D₁₀, D₅₀, and D₉₀ values are useful ways for indicating a particle size distribution. D₉₀ refers to the value for the particle size for which at least 90 volume percent of the particles have a size smaller than the value. Likewise D₅₀ and D₁₀ refer to the values for the particle size for which 50 volume percent, and 10 volume percent, of the particles have a size smaller than the value. Methods for determining D₁₀, D₅₀, and D₉₀ include laser light diffraction, such as using equipment sold by Malvern Instruments Ltd. of Malvern, Worcestershire, United Kingdom.

In an embodiment, celecoxib obtained according to process of the present application have a mean particle size of less than about 50 µm, D₁₀ less than 10 µm or less than 5 µm, D₅₀ less than 20 µm or less than 30 µm, and D₉₀ less than 50 µm or less than 25 µm

### DEFINITIONS

The following definitions are used in connection with the present application unless the context indicates otherwise.

An "alcoholic solvent" is an organic solvent containing a carbon bound to a hydroxyl group. "Alcoholic solvents" include, but are not limited to, methanol, ethanol, 2-nitroethanol, 2-fluoroethanol, 2,2,2-trifluoroethanol, hexafluoroisopropyl alcohol, ethylene glycol, 1-propanol, 2-propanol (isopropyl alcohol), 2-methoxyethanol, 1-butanol, 2-butanol, i-butyl alcohol, t-butyl alcohol, 2-ethoxyethanol, diethylene glycol, 1-, 2-, or 3-pentanol, neo-pentyl alcohol, t-pentyl alcohol, diethylene glycol monomethyl ether, diethylene glycol monoethyl ether, cyclohexanol, benzyl alcohol, phenol, glycerol, C₁₋₆alcohols, or the like,

The phrase "'substantially free" as used herein, unless otherwise defined, means comprising less than about 5%, less than about 2%, less than about 1 %, less than about 0.5%, less than about 0.3%, less than about 0.1%, or less than about 0.05% by weight, of crystalline Forms I or Form 11 in the crystalline Form III.

The term "substantially free" for either crystalline Form I or Form II, or both, in crystalline Form III of celecoxib obtained in the present application indicates that the contents of those forms are below the limits of detection in a powder X-ray diffraction analysis.

The term 'substantially free' with respect to Form I refers to the presence of Form I in Form III below the limit of detection ("LOD") as determined using a powder X-ray diffractometer. The LOD for Form I is about 1.5% (w/w) of Form I in crystalline Form III of celecoxib. The term 'substantially free' with respect to Form 11 refers to the presence of Form II in Form III celecoxib below the limit of detection (LOD) as determined by the absence of a characteristic powder X-ray diffraction peak based on signal to noise ("S/N") ratio.

In the absence of physical sample of a polymorphic form, the S/N ratio method is effective to determine the presence of a characteristic peak as an impurity in another form. With the unavailability of pure Form II of celecoxib, the S/N method is used to determine the content of Form II impurity with respect to the presence of characteristic peak in the sample of Form III. If the signal to noise ratio is less than 3, then the impurity in the batch sample is below the detection limit. If the signal to noise ratio is equal to or more than 3, then that impurity in the batch sample is above the limit of detection.

Certain specific aspects and embodiments of the present application will be explained in more detail with reference to the following examples, which are provided only for purposes of illustration and should not be construed as limiting the scope of the application in any manner. Reasonable variations of the described procedures are intended to be within the scope of the present application.

### EXAMPLES

**EXAMPLE 1: PREPARATION OF CRYSTALLINE FORM III OF CELECOXIB USING METHANOL AND WATER.** Celecoxib (100 g) and methanol (1800 mL) are placed into a round bottom flask and stirred to form a solution. Activated carbon (5 g) is added, stirred for 10-15 minutes, and filtered. The solid is washed with methanol (200 mL). The combined filtrate is placed into a round bottom flask and water (2000 mL) is added slowly in about 30-45 minutes. The mixture is maintained at 25-35°C for about 3-5 hours. The formed solid is collected by filtration, washed with water (200 mL), and dried under suction. The wet solid is dried at 60-65°C under vacuum. The wet solid is dried at 60-65°C under vacuum. The solid was subjected to wet milling, Particle size distribution: 27.95 micrometers

**EXAMPLE 2: PREPARATION OF CRYSTALLINE FORM III OF CELECOXIB USING ACETONITRILE AND WATER.** Celecoxib (10 g) and acetonitrile (40 mL) are placed into a round bottom flask and stirred for complete dissolution. Activated carbon (1 g) is added, stirred for about 15 minutes, and filtered. The filtrate is placed into a round bottom flask and water (200 mL) is added slowly in about 30 minutes. The mixture is maintained at 25-35°C for about 40 minutes. The formed solid is filtered, washed with water (80 mL), and dried under suction. The wet solid is dried at 60-65°C under vacuum.

**EXAMPLE 3: WET MILLING OF CELECOXIB FORM III:** Celecoxib (50 g) and methanol (1000 mL) are placed into a round bottom flask and water (1000 mL) was added to it. The slurry was subjected to wet milling. The slurry was then filtered, and the wet solid dried at 60-65°C under vacuum to yield 32 g of the title compound. Particle size distribution: D₉₀: 23 µm.

Throughout this application, various publications are referenced. The disclosures of these publications in their entireties are hereby incorporated by reference into this application in order to more fully describe the state of the art as known to those skilled therein as of the date of the application described and claimed herein.

While particular embodiments of the present application have been illustrated and described, it would be obvious to those skilled in the art that various other changes and modifications can be made without departing from the spirit and scope of the application. It is therefore intended to cover in the appended claims all such changes and modifications that are within the scope of this invention.

## Claims

1. A process for the preparation of crystalline Form III of celecoxib substantially free of polymorphic Forms I and II comprising:
a) providing a solution of celecoxib in an alcoholic or a nitrile solvent;
b) combining the solution of celecoxib with water; and
c) isolating pure crystalline Form III of celecoxib.

2. The process of claim 1, wherein the alcoholic solvent is methanol, ethanol, 2-propanol, 1-butanol, 1 -propanol, or tertiary butyl alcohol.

3. The process of claim 2 wherein the alcoholic solvent is methanol.

4. The process of claim 1 wherein the nitrile solvent is acetonitrile or propionitrile,

5. The process of claim 4, wherein the nitrile solvent is acetonitrile.

6. A process for preparation of crystalline Form III of celecoxib substantially free of crystalline forms I and II comprising wet milling a sample of crystalline Form III of celecoxib.

7. Crystalline Form III of celecoxib substantially free from crystalline Forms I and II.

8. Crystalline Form III of celecoxib of claim 7, wherein the content of Form I is less than 1.5% as determined by X-ray diffractometer and the content of Form II is below the limits of detection as determined by S/N ratio method.

9. Crystalline Form III of celecoxib of claim 7, wherein the content of Form I is less than about 1.5% as determined by X-ray diffractometer.

10. Crystalline Form III of celecoxib of claim 7, wherein the content of Form II is below the limits of detection as determined by the S/N ratio method.

11. Crystalline Form III of celecoxib of claim 7 with a PXRD pattern substantially as depicted in Fig. 1.
